# EUROPEAN PATENT APPLICATION

(11) **EP 4 109 456 A1**
(43) Date of publication of application: **28.12.2022**
(21) Application number: 21181919.8
(22) Date of filing: 27.06.2021
(51) Int. Cl.: G16C 60/00

(54) **METHODOLOGY FOR DEVELOPING SUSTAINABLE ALUMINIUM PRODUCTS**

(71) Applicant: Norsk Hydro ASA, 0240 Oslo (NO)
(72) Inventor: Furu, Trond, 6600 Sunndalsøra (NO); Myhr, Ole Runar, 2830 Raufoss (NO)
(74) Representative: Hydro IPD

(57) **Abstract**

The present invention relates to mixing raw materials from two or more aluminium metal sources from a Metal Base. The raw material is categorized and stored in a database from where candidate alloys are randomly proposed by a computer and each single Candidate alloy entity is categorized. Candidate alloys having a CO2 index that is above a set threshold can be discarded for further evaluation. The remaining candidate alloys are further evaluated and qualified with regard to their ability to fulfill the actual functions of use for instance as a specific product and followingly a set of Qualified Candiate alloys can be defined.

## Description

The present invention relates to developing sustainable aluminium products by applying a computer-implemented method for generating candidate aluminium alloy entities for a particular use, based upon mixing raw material from two or more aluminium metal sources and simulation of properties to qualify candidate aluminium alloy entities in accordance with intended use.

The candidate aluminium alloys qualified by the simulation can be applied for production of aluminium products, where the alloy complies with certain physical requirements such as mechanical strength including yield- and ultimate tensile strength, electrical- and thermal conductivity, corrosion resistance and more, ductility, fatigue properties, fracture toughness, grain structure, and surface appearance in terms of gloss.

In prior art there are many sources describing the effects of applying alloying elements in aluminium, effects of tempering and hardening, description of the aluminium classification system and appropriate use of various alloy such as;
"https://en.wikipedia.org/wiki/Aluminium alloy"

The abovementioned reference can provide for a general basic introduction in various alloying series and their application and also various hardening/tempering techniques.

Further, as a part of the state of the art there are books such as:
"Joseph R. Davis; Alloying: Understanding the Basics, ASM International, 2001, Pages: 647, ISBN: 978-0-87170-744-4"

This reference relates to the influence of alloy additions on various properties as mechanical properties and physical properties, behaviour related to corrosion, and chemical behaviour, and processing and manufacturing characteristics. The reference also describes relevant beneficial effects of major alloy additions, grain refiners, and other elements that have been deliberately added to improve performance and also detrimental effects of minor elements.

There are also software programs known in the art that have been developed to predict various physical and chemical parameters of aluminium alloys:
Alstruc: The microstructure evolution during casting and homogenisation is calculated by Alstruc. The required input to this model includes chemical composition of the alloy, as well as thermal history during casting. If the latter is not known, Alstruc makes an estimate based on available process data for the casting, like billet diameter and casting speed. In the solidification module of Alstruc, the metal is assumed to solidify gradually with the concentration of each alloying element, given from experimental phase diagram data. The microstructure outputs from the solidification module are input to the homogenization module of Alstruc, which calculates the diffusion controlled levelling out of concentration gradients, as well as possible changes of the existing particle structure due to phase transformations and diffusion controlled reactions, i.e., growth, dissolution, and coarsening. In addition, the program predicts the formation of dispersoids, which are fine particles that may precipitate from the solid solution during the homogenization cycle if the matrix contains an excess amount of elements compared with the equilibrium concentrations. The outputs from Alstruc can be used as inputs to other computer programs that calculate product properties, e.g. the software programs Alsoft and NaMo described below.
(Dons, A.L., Jensen, E.K., Langsrud, Y., Trømborg, E., and Brusethaug, S., Metallurgical and Materials Transactions, Vol. 30A, 1999, p. 2135; Dons, A.L., Journal of Light Metals, Vol. 1, 2001, p. 133).
Alsoft is a software program that calculates the grain structure in terms of fraction recrystallized grains, and corresponding grain size, The Alsoft model is an analytical, statistical approach to predict the combined effect of static recovery and recrystallization during annealing of deformed aluminium. The outputs from Alsoft, i.e. parameters that describe the grain structure of the product, can be used to estimate several different properties that depend on the grain structure when combined with other software programs, e.g. corrosion resistance, surface appearance, and fatigue properties. (T.Furu, K.Marthinsen and E.Nes: Materials Science and Technology, 6, 1093 (1990) H.E.Vatne, T.Furu, R.Ørsund and E.Nes, Acta Met. Mater. Vol. 44, pp4463-4473, (1996))
NaMo is a software program that calculates the yield strength, the ultimate tensile strength, and the elongation to necking for alloy, as well as electrical conductivity. Inputs to the model include the alloy composition as well as a specified heat treatment including heating- and cooling rates. NaMo is physically based and calculates the evolution of nano-structure size precipitates and converts extracted key nano-structure parameters to a corresponding stress-strain curve based on dislocation theory. The outputs from NaMo can be used as inputs to other software programs to calculate a range of properties of the final products, including thermal stability, energy absorption during loading, and load bearing capacity of welded structures.
(Myhr, O.R., Grong, Ø., and Schafer, C., Metall. Mater. Trans A, Vol. 46A, 2015, p. 6018; Myhr, O.R., Grong, Ø., and Pedersen, K.O., Metall. Mater. Trans A, Vol. 41A, 2010, p. 2276).

In principle, the invention can be relevant for designing several aluminium products and properties of same. The products can be made by extrusion, rolling, forging, castings or additive manufacturing. The products may be composed and made by joining and welding.

In particular, the invention relates to minimizing CO2 footprint in production and by final design of aluminium products, by use of a computer based stochastic method for providing candidate aluminium alloys from a pre-defined set of aluminium metal sources, and qualifying aluminium alloys among said candidate alloys with regard to certain properties by computer based simulation and selection.

In accordance with the method, it is calculated a CO2 index for each candidate alloy entity stemming from a randomized mixture of raw aluminium metal sources, based upon CO2 indexes representative for the various aluminium metal sources.

In general, the simulations are carried out by a program installed on a computer, where the computer is provided with one or more interfaces for a user.

Based upon the calculated CO2 index for a digitally provided candidate alloy, the CO2 footprint related to the production of the product can be calculated.

The ambitious climate goals set for lowering CO2 emissions encourage businesses to join efforts in bringing the level of emissions down.

A main focus behind the present invention is therefore to provide a holistic approach to the CO2 footprint, where significant operations in the overall process chain for producing the aluminium product can be simulated by a model run by a program on a computer.

These operations can be performed according to several sets of operational parameters as well as selecting source of the metal and various properties of the aluminium alloy to be able to establish the desired/acceptable total CO2 footprint of alternative/different operations/alloys.

The invention is based upon a "Through Process Modelling" principle where computer-implemented simulations provides alternatives for producing aluminium products compatible with material properties according to a defined specification of the final product and with a specified, low CO2 footprint.

The invention is further related to products of aluminium alloys that are manufactured in several operations. The operations are linked in a process chain that can comprise some main operations such as producing molten metal from a mix of sources, melt treatment, such as purification and chemistry/alloy adjustment (i.e., adjustment of the chemical composition of the alloy), casting, extrusion, and shaping. In the design of the alloy, compensation metallurgy may be applied.

According to the present invention a computer-implemented method is applied in a novel and inventive context for simulating the CO2 footprint present by various raw materials, by interlinked operations of a process chain, all the way from the source of aluminium metal to the finished aluminium alloy product.

One advantage with the present invention is that the CPU time consumed for establishing qualified candidate alloys can be very low by starting with selection on basis of the CO2 index and setting that at the appropriate level upfront in the simulations. That will say setting a level for discarding those candidate alloys with a CO2 index above a set threshold from a first set of candidate alloys. It may take milli seconds to generate candidate alloys on basis of the CO2 index based upon selected raw materials compared to simulating the physical / chemical properties of the candidate alloys. Therefore, the simulations and corresponding selections should in an early phase discard the candidate alloys having too high CO2 index, and generate a second set of candidate alloys.

The invention is defined by the features as defined in the independent claim 1.

Preferred embodiments of the invention are defined by the features as given in the dependent claims 2-10.

The present invention will be described in further detail in the following by way of examples and with reference to the drawings, where:
Fig. 1 discloses in its upper part a Metal Source scheme of Raw materials with specified alloy composition and CO2 index, and below that a scheme of randomly generated "Candidate alloys", while the lower part of the Figure discloses a flow chart of a data program run on a computer for selection of alloys from the list of "Candidate alloys",
Fig. 2 discloses a more detailed outline of Figure 1, where the upper table shows Raw materials with index i, which are classified according to their fraction of electrolysis metal (F_{E}), fraction Post Consumed Scrap (F_{PCS}), and fraction Process Scrap (F_{PS}), while the lower table in Figure shows examples on various candidate alloys obtained from mixing the raw materials,
Fig. 3 discloses geometry assumptions and load case assumptions for an example further described in Figures 4, 5, 6, 7, and 8,
Fig. 4 discloses a table that shows composition and selected parameters for two different alloys named Alloy A and Alloy B,
Fig. 5 discloses two different cross-sections which give the same bending capacity, since alloy A has a lower second moment of area Iₓ, but a higher yield stress than alloy B.
Fig. 6 discloses a Flow-chart that illustrates how a cross-section for alloy j is calculated by an iteration procedure using an optimisation software,
Fig. 7 discloses a schematic diagram illustrating different ram speeds during extrusion of two different profiles (alloys),
Fig. 8 discloses Schematic diagram illustrating use "compensational metallurgy".

In the following, some terms and definitions related to the processing of the alloys applied in this document are given:

### Definitions Raw material alloys

For the Raw material "R" the following applies, with suffix "R":

| | | |
|---|---|---|
| Entity | 1 "R" | - means alloy 1 "R" |
| Data field | 1 "R" | - means CO2 index "R" |
| Data field | 2 "R" | - means chemistry "R" |
| Record | 1 "R" | - means data for CO2 index and chemistry for Entity1 "R" |
| | | |
| Entity | 2 "R" | - means alloy 2 "R" |
| Data field | 1 "R" | - means CO2 index "R" |
| Data field | 2 "R" | - means chemistry "R" |
| Record | 2 "R" | - means data for CO2 index and chemistry for Entity 2 "R" |
| | | |
| Entity | n "R" | - means alloy n "R" |
| Data field | 1 "R" | - means CO2 index "R" |
| Data field | 2 "R" | - means chemistry "R" |
| Record | n "R" | - means data for CO2 index and chemistry for Entity n "R" |
| | | |
| One file | "R" | -contains Record 1 "R", Record 2 "R" ... Record n "R" |

### Definitions Candiate alloys

For the Candidate alloys "C" the following applies, where a suffix "C" is introduced:

| | | |
|---|---|---|
| Entity | 1 "C" | - means alloy 1 "C" |
| Data field | 1 "C" | - means CO2 index "C" |
| Data field | 2 "C" | - means chemistry "C" |
| Record | 1 "C" | - means data for CO2 index and chemistry for Entity1 "C" |
| Entity | 2 "C" | - means alloy 2 "C" |
| Data field | 1 "C" | - means CO2 index "C" |
| Data field | 2 "C" | - means chemistry "C" |
| Record | 2 "C" | - means data for CO2 index and chemistry for Entity 2 "C" |
| | | |
| Entity | m "C" | - means alloy m "C" |
| Data field | 1 "C" | - means CO2 index "C" |
| Data field | 2 "C" | - means chemistry "C" |
| Record | m "C" | - means data for CO2 index and chemistry for Entity m "C" |
| | | |
| One file | "C" | -contains Record 1 "C", Record 2 "C" ... Record m "C" |

### Definitions Qualified Candiate alloys

For the Qualified Candidate alloys "QC" the following applies, where a suffix "QC" is introduced:

| | | |
|---|---|---|
| Entity | 1 "QC" | - means alloy 1 "QC" |
| Data field | 1 "QC" | - means CO2 index "QC" |
| Data field | 2 "QC" | - means chemistry "QC" |
| Record | 1 "QC" | - means data for CO2 index and chemistry for Entity1 "QC" |
| | | |
| Entity | 2 "QC" | - means alloy 2 "QC" |
| Data field | 1 "QC" | - means CO2 index "QC" |
| Data field | 2 "QC" | - means chemistry "QC" |
| Record | 2 "QC" | - means data for CO2 index and chemistry for Entity 2 "QC" |
| | | |
| Entity | m "QC" | - means alloy m "QC" |
| Data field | 1 "QC" | - means CO2 index "QC" |
| Data field | 2 "QC" | - means chemistry "QC" |
| Record | m "QC" | - means data for CO2 index and chemistry for Entity m "QC" |
| One file | "QC" | -contains Record 1 "QC", Record 2 "QC" ... Record m "QC" |

### Detailed description of the invention

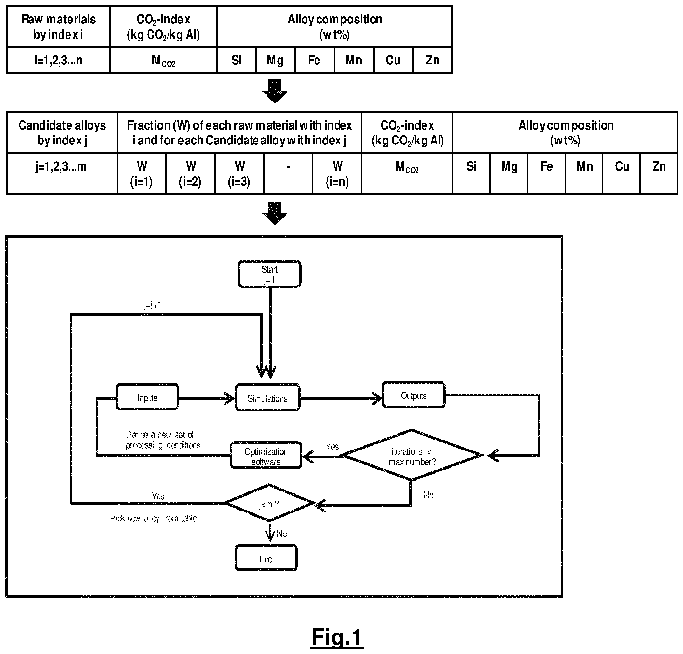

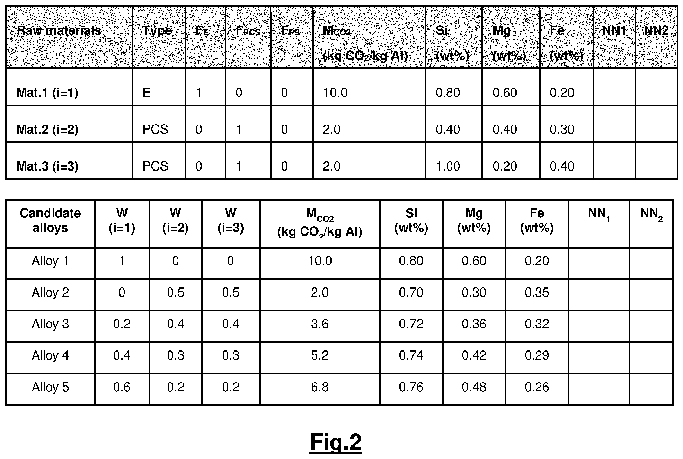

In the upper part of Fig. 1 there is shown a Metal Source scheme of Raw materials with specified alloy composition and CO₂ -index. Below the upper part it further discloses a scheme of randomly generated "Candidate alloys" from a mix of alloys from the Metal Source scheme, the alloy objects has a calculated chemical composition and CO₂ -index, see also Fig. 2, The lower part of the Figure discloses a flow chart of a data program run on a computer for selection of alloys from the list of "Candidate alloys" that falls within predefined criterions such as physical or chemical properties that are entered manually in the computer for establishing a list of "acceptable alloys",

A more detailed outline of Figure 1 is shown in Fig 2. The upper table shows raw materials with index i, which are classified according to their fraction of electrolysis metal (F_{E}), fraction Post Consumed Scrap (F_{PCS}), and fraction Process Scrap (F_{PS}). The raw materials usually belong to one of these three categories, and therefore the fractions are either 0 or 1 in the table, even though mixtures of fractions may be possible. Each raw material with index i, is associated with a given CO₂-index, as shown by one of the columns. This can be calculated based on the production history of the raw material. In the table, the one with the highest CO₂-index is the raw material with F_{E}=1, which is typical. The second table in Figure 2 shows examples on various candidate alloys obtained from mixing the raw materials Mat. 1, Mat. 2, and Mat. 3 in various fractions, as described by W(i=1), W(i=2), and W(i=3). The resulting CO₂-index shown in the table, is given from the weight fractions W. Furthermore, the resulting alloy composition for Si, Mg, Fe, and other elements NN1, NN2 etc. are also obtained from the weight fractions W(i=1), W(i=2), and W(i=3).

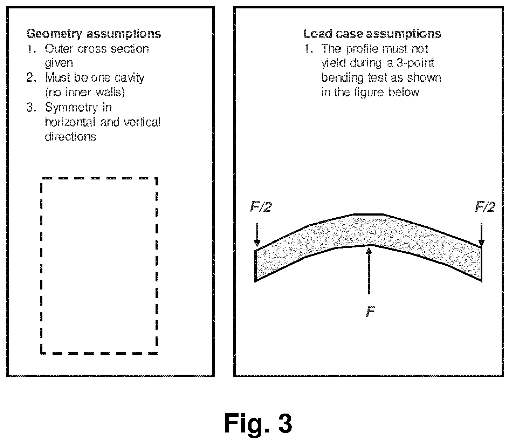

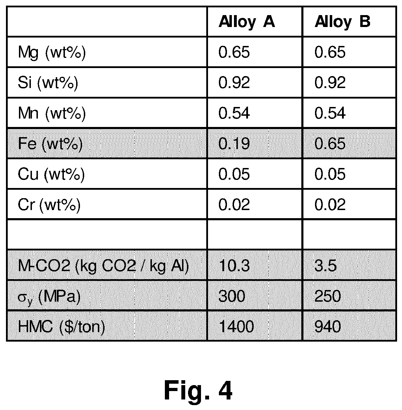

Fig. 3 discloses geometry assumptions and load case assumptions for the example described in Figures 4, 5, 6, 7, and 8.

The table in Fig. 4 shows composition and selected parameters for two different alloys named Alloy A and Alloy B. The alloy composition is similar, except for Fe, which is 0.19wt% for alloy A, and 0.65wt% for alloy B. The alloys are produced from different raw-material sources, giving different CO₂-index (i.e. 10.3 for alloy A, and 3.5 for alloy B). This is because a high Fe-content, as for alloy B, allows for a corresponding high fraction of Post Consumed Scrap to be used. The resulting yield stress is higher for Alloy A than Alloy B, since Fe has a detrimental effect on the yield stress for this type of alloys since it ties up Si in coarse non-hardening particles. The Hot Metal Cost (HMC) is lower for alloy B than alloy A, since a high Fe-content allows a high fraction of post consumed scrap to be used as raw material base, which has in general a lower price than electrolysis metal.

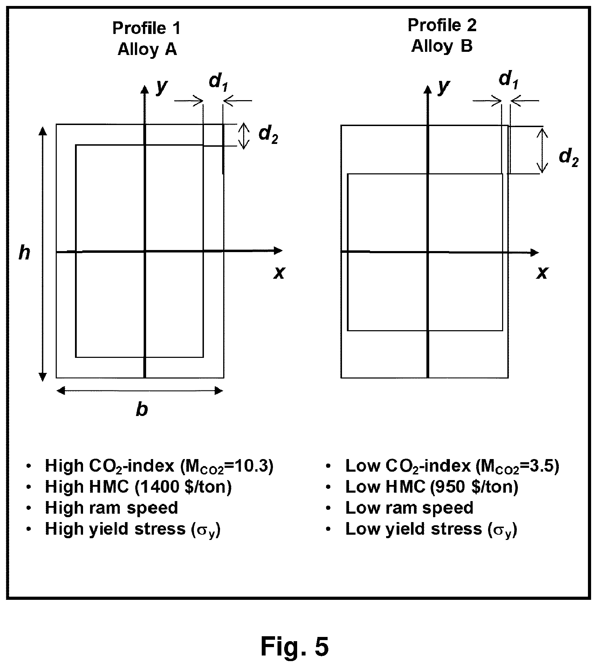

Fig. 5 discloses two different cross-sections which give the same bending capacity, since alloy A has a lower second moment of area Iₓ, but a higher yield stress than alloy B.

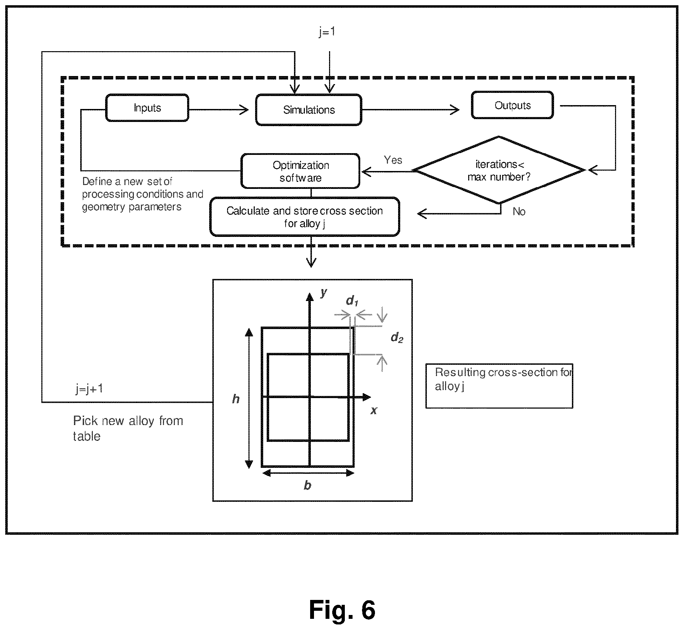

In Fig. 6 there is disclosed a Flow-chart that illustrates how a cross-section for alloy j is calculated by an iteration procedure using an optimisation software. In this example the bending capacity of the extrusion is assumed to be critical in the sense that the profile must carry a defined bending moment before yielding occurs at the upper and/or lower surface. This critical bending moment depends on the stiffness of the profile through the second moment of area Iₓ and Youngs modulus E, where E can be regarded as constant for aluminium alloys. The critical bending moment also depends on the yield stress σ_{y}. Hence, a profile with a high σ_{y} and a low Iₓ may give the same bending capacity as a profile with a low σ_{y} and a high Iₓ. In the example, it is assumed that Iₓ can be calculated from a given profile geometry, which is simple for the hollow rectangular profile shown in Fig. 6., since analytical solutions from basic beam theory can be applied. The calculations of Iₓ must be done automatically for each new profile geometry that is tested. As a starting point, an alloy is selected from the candidate alloy database. The first time, this corresponds to j=1, and initial set of inputs are given for these simulations. The simulations may include various models for predictions of properties, e.g. the models described previously (Alstruc, Alsoft, and NaMo) or similar. According to the flow-chart in Fig. 6, simulations are carried out producing outputs, which are evaluated by the optimisation software. This software is responsible for the dataflow shown in the flow-chart. The optimisation software is also responsible for selecting the inputs for the next series of simulations, where new processing conditions are defined. This gives a new set of outputs, which again are evaluated by the optimisation software. This procedure continues until the number of iterations reach a pre-defined maximum value, or some other criteria is obtained as defined by the user. When the maximum iterations are achieved, the resulting processing conditions, and resulting properties are stored together with parameters that define the profile geometry. In the present example, this will be the parameters d₁ and d₂, since h and b are constant. As shown in the flow-chart, the next step is now to pick a new candidate alloy from the database, corresponding to j=j+1. The procedure described above is then repeated, resulting in a new profile geometry, and corresponding processing and property values. The algorithm described above and outlined by the flow-chart in Fig. 6, gives as a result a set of cross section geometries with associated processing conditions and properties, which all satisfy requirements on CO2-index, since only candidate alloys with acceptable CO2 index are considered.

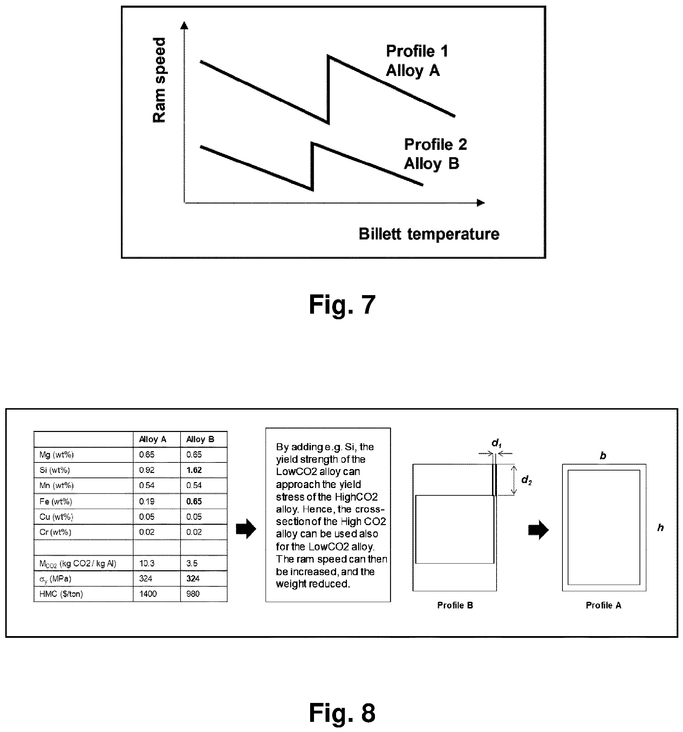

Fig. 7 shows a schematic diagram illustrating that the two different alloy cross-sections shown in Figure 5 must be processed with different ram speed during extrusion, since the thin vertical walls in Profile 2 (Alloy B) is more susceptible for surface tearing than the uniform wall thickness for Profile 1 (Alloy A).

Fig. 8 shows a schematic diagram illustrating how "compensational metallurgy" can be used to keep profile geometry A for the alloy with high Fe-content, by adding Si which as a result may give the same yield stress as Alloy A.

According to one aspect of the invention, there is as a starting point defined an aluminium metal base of pre-defined metal sources such as post consumed scrap (PCS) (bin 1, 2 ,3..), primary metal (PM) (electricity based on fossil fuels like coal, or electricity based on hydroelectric power), production scrap (PS) (bin 1, 2, 3), and where each source has a known/analysed chemical composition (alloy) and a CO2 index allotted thereto, and this information is stored as records in a datafile that is stored on a recordable medium. The records comprise information of each Raw material alloy entity 1,2... n "R", where one data field represents the CO2 index, and a second data field represents the chemical composition.

According to one aspect of the invention, a first process/operation is carried out on a computer with a reader for a recordable medium and a program reading from the recordable medium and where a randomly selected weight fraction mixture of entities "R" from the said metal sources is processed by the computer and the chemical composition and a CO2 index for each mixture is calculated and a set of new Candidate alloy entities "C" with given chemical composition and CO2 index are generated and stored as entity 1 "C", entity 2 "C",.... entity m "C" as a file on a recordable medium. By this it is generated a file with a new set of candidate "C" entities, each having a specific chemical composition and CO2 index.

According to one aspect of the invention, there is manually selected an upper limit for the CO2 index, where those alloys (entities) above that limit will be excluded for further processing in a following simulation process/operation. The selection of the said limit can be entered manually by a user's interface via a keyboard or the similar connected to the computer, where the limit value is loaded in the computer running the program. This can be done before or after running the randomly selection mentioned above.

According to one aspect of the invention, candidate entities below the upper limit set for the CO2 index are acceptable and processed as a set of candidate Records "C" 1, 2 ... m with corresponding data fields in a simulator that comprises a computer and a program that is able to simulate specific properties of the candidate entities i.e. aluminium alloys of various chemical compositions. The simulated properties can be one or more of yield- and ultimate tensile strength, ductility, electrical and thermal conductivity, corrosion resistance, fatigue properties, fracture toughness, grain structure, and surface appearance in terms of gloss and more.

According to one aspect of the invention, the simulated properties for each candidate entity Record "C" 1, 2 ...m can be presented in a table together with the corresponding calculated CO2 index and with the chemistry thereof. The simulated properties are added as additional data fields for the individual entity "C" records 1, 2 ...m

In one aspect of the invention, the above mentioned set of data is further processed where a threshold for one or more of the aforementioned properties in the set of data can be entered manually by an user's interface via a keyboard or the similar connected to the computer where the threshold value(s) is lodged in the program. Such threshold values/parameters are considered as decisive criterions set for the final product.

In one aspect of the invention a selection of entities (alloys) based upon a threshold value is performed, where the item(s) not fulfilling the criterion set are removed from the set of data and the remaining items being qualified "QC" and their corresponding data are stored on a recordable medium for further processing.

In one aspect of the invention the user will at this stage have from 0 up to < m candidate entities (alloys) that fulfil the criterions set (i.e. upper limit for the CO2 index and property limit).

In one aspect of the invention, a further selection step may involve that an even lower CO2 index can be selected and entered into the computer by a user, if the number of candidate entities is large.

In one aspect of the invention, if the number of candidate entities is small/zero, a redesign of the final product may be considered. In particular if the final product has some design freedom with regard to its geometrical shape and the strength of the proposed alloy(-s) are too low.

In one example of the invention, an overall simulation process for making an acceptable product such as an extruded profile from metal sources can be illustrated by various steps:
- Input definition: Product to be produced (shape limitations, in particular outer shape, minimum strength, yield, corrosion resistance etc.)
- Aluminium Raw metal with known chemistry of its various Aluminium Metal sources (post consumed scrap, process scrap, primary metal) represented by raw metal alloy entities with known chemistry and CO2 index stored as entities in a database.
- Generate in a stochastic manner Candidate entity alloys from said Aluminium Metal Sources and generating a Database of Candidate aluminium alloy entities with known CO2 index;
- Discarding Candidate entity(-ies) with too high CO2 index
- Candidate alloy entity qualification (verification for instance with regard to pre-defined physical and chemical parameters and thermal treatment of the alloy)
- Re-design of final product for allowing more candidate alloys entities. Threshold for minimum strength or freedom to re-design structural part
- Verifying Extrudability of selected alloy, press speed threshold
- CO2 footprint calculations, in manufacturing process, product in use

Regarding step Re-design of final product of Fig 4-6:
The design of the profile may be fixed if the customer requires a specific, detailed geometry. Alternatively, the design may be optimised, which usually requires that some characteristic dimensions of the cross section are parameterized in the computer simulations, which are typically Finite Element (FE) simulations. The geometry affects a range of properties. Examples of properties that are directly related to the profile design includes the stiffness which depends on the moment of inertia as well as the axial load bearing capacity, which is proportional to the area.

Mechanical properties: Examples of customer specified mechanical properties are tensile properties, which may be specified as follows:
- Yield stress>190MPa
- Ultimate tensile stress>215MPa
- Elongation to fracture>15%

## Claims

1. A computer-implemented method for simulation of properties of aluminium alloys for producing an aluminium product with respect to product relevant physical / chemical parameters and with a CO2 index below a pre-defined limit wherein the method comprising;
a) establishing an aluminium Metal Base of pre-defined metal sources such as post consumed scrap, primary metal, production scrap, and where each source is defined as a Raw metal entity (R1..n) and stored on a recordable medium, the entities further having datafields for chemical composition and the CO2 index allotted thereto,
b) entering the information from step (a) in a data program provided for generating stochastically a plurality of candidate alloy entities with calculated chemistry and a calculated CO2 index for each alloy entity based upon a weight fraction mix of the metal sources in the said aluminium Metal Base, the candidate alloy entities and their properties are stored on a recordable medium, -and where a threshold for max CO2 index is defined and candidate alloy entities above said threshold are discarded from further processing,
c) entering the data from step (b) into a data program being able to simulate physical (mechanical) property parameters and/or chemical parameters such as strength, corrosion resistance, electrical conductivity of each candidate alloy entity, and running the program in an iterative process,
d) and further comparing the output of simulations of step c) with pre-defined property parameter thresholds (limits) and select those being compliant to produce a set of qualified alloy candidates, and producing an output that is stored on a recordable medium as datafile(s).

2. A method according to claim 1,
wherein,
threshold values as max CO2 index, min strength, min corrosion resistance, min electrical conductivity can be entered in the computer program via a keyboard or the similar by a manual interaction.

3. A method according to claim 1,
wherein,
the simulation relates to an alloy for producing an extruded aluminium product and further including a model for extrudability (press speed) of the proposed alloys either in step c) or the qualified alloys of step d), the model is converted into a respective data program that is installed on the computer where the program has a predefined set of data for extrudability of all alloys that can be output from step (c) or (d) wherein the extrusion speed of each of the simulated alloys can be calculated and a set of output data is produced.

4. A method for simulation of a process for producing an extruded aluminium product according to claim 3,
wherein,
the program has a threshold value for press speed that can be predefined, where alloy compositions of a press speed below said threshold can be discarded in the iteration process.

5. A method for simulation of a process for producing an extruded aluminium product according to claim 3,
wherein,
the program has a predefined threshold value for the strength of the final product, where alloy compositions of a strength value below said threshold can be discarded in the recurring process.

6. A method for simulation of a process for producing an extruded aluminium product according to claim 3,
wherein,
the program has a predefined threshold value for the corrosion resistance of the final product, where alloy compositions of a value below said threshold can be discarded in the recurring process.

7. A method for simulation of a process for producing an extruded aluminium product according to claim 3,
wherein,
the program has a predefined threshold value for the electrical conductivity of the final product, where alloy compositions of a value below said threshold can be discarded in the recurring process

8. A method according to claim 1,
wherein,
the simulation relates to an alloy for producing a forging stock aluminium product and further including a model for thermo mechanic processing of the candidate alloys either in step c) or the qualified alloys of step d), the model is converted into a respective data program that is installed on the computer where the program has a predefined set of data for thermo mechanic processing of all alloys that can be output from step (c) or (d) wherein the parameters as fatigue properties and stiffness of each of the simulated alloys can be calculated and a set of output data is produced.

9. A method according to claim 1,
wherein,
the simulation relates to an alloy for producing a hollow beam aluminium product that has a predefined set of parameter(s) related to geometry and strength, in particular yield strength, and further including a model for processing of the candidate alloys from step c), or the qualified candidate alloys from step d), where the model is converted into a respective data program that is installed on the computer where the program has a predefined set of geometry data for several cross-sections, where for each alloy the resulting strength of the beam is calculated by an iteration procedure using an optimisation software for each cross-section, where for each of said alloys resulting processing conditions and resulting strength are stored together with parameters that define each of the profile geometries.

10. A method according to claim 9,
wherein a threshold for min strength of the beam is defined, and and alloys not complying with this requirement are discarded.
